# EUROPEAN PATENT SPECIFICATION

(11) **EP 3 747 417 B1**
(45) Date of publication and mention of the grant of the patent: **30.08.2023**
(21) Application number: 19748443.9
(22) Date of filing: 01.02.2019
(51) Int. Cl.: A61F 13/532

(54) **ABSORBENT BODY, METHOD FOR MANUFACTURING SAME, AND ABSORBENT ARTICLE**
ABSORBIERENDER KÖRPER, VERFAHREN ZU SEINER HERSTELLUNG UND ABSORBIERENDER ARTIKEL
CORPS ABSORBANT, SON PROCÉDÉ DE FABRICATION, ET ARTICLE ABSORBANT

(30) Priority: 02.02.2018 JP 2018017005
(43) Date of publication of application: 09.12.2020
(73) Proprietor: DAIO PAPER CORPORATION, Shikokuchuo-shi Ehime 799-0492 (JP)
(72) Inventor: KOYAMA, Hidetoshi, Sakura-shi, Tochigi 329-1411 (JP)
(74) Representative: Bandpay & Greuter
(86) International application number: PCT/JP2019/003551
(87) International publication number: WO 2019/151464

(56) References cited:
- JP-A- H1 014 978
- JP-A- S6 129 350
- JP-A- H03 502 531
- JP-A- H09 276 391
- JP-A- 2006 297 073
- JP-A- 2007 130 818
- JP-A- 2012 115 295
- JP-A- 2012 254 329
- US-A- 5 447 677
- US-A1- 2013 165 882

## Description

### Technical Field

The present invention relates to an absorbent body mainly used for absorbent articles such as incontinence pads, disposable diapers, sanitary napkins, and vaginal discharge sheets, and relates to an absorbent body excellent in liquid diffusion property without causing gel blocking even when a content ratio of highly water-absorbent polymers is increased, a method for manufacturing the same, and an absorbent article.

### Background Art

Conventionally, as the absorbent article, there has been known an absorbent article in which an absorbent body made of cotton-like pulp, a highly water-absorbent polymer, etc. is interposed between a leak-proof sheet such as a polyethylene sheet or a polyethylene sheet laminated nonwoven fabric and a liquid-permeable surface sheet such as a nonwoven fabric or a liquid-permeable plastic sheet.

As a method for manufacturing the absorbent article, there is a method in which a belt-shaped carrier sheet is continuously supplied to a deposition apparatus including a particle suction unit, particles are supplied in a scattered state on the carrier sheet, the particles are sucked from the particle suction unit and deposited on the carrier sheet, and a particle fiber stack including the carrier sheet and the particles is obtained (see Patent Documents 1 and 2 below).

By using such a method for manufacturing an absorbent body, it is possible to increase a content ratio of highly water-absorbent polymers to continuously produce a thin absorbent body having a low basis weight of pulp and a high water absorption capacity at high speed. In this instance, there is an advantage that the yield of the highly water-absorbent polymers can be improved.

Patent Document 3 describes an apparatus and method for producing absorbent cores intended to reduce the problem of gel blocking. A first layer consisting of a first absorbent material is formed, then a second layer comprising a mixture of the first absorbent material with a second absorbent material is deposited on top.

### Citation List

### Patent Document

Patent Document 1: JP-A-2001-171029
Patent Document 2: JP-A-2008-507384
Patent Document 3: US 5,447,677

### Summary of the Invention

### Technical Problem

However, in the manufacturing methods described in Patent Documents 1 and 2, since the content ratio of the highly water-absorbent polymers is increased, when repeated urination occurs at intervals, so-called "gel blocking" tends to occur, in which voids between the polymer particles swollen by water absorption during previous urination are extremely reduced, and there is concern that the required water absorption capacity, liquid diffusion property, and absorption speed may not be exhibited. As a result, urine permeation is inhibited by bonding between the polymer particles, and a phenomenon that urine whose permeation is inhibited reverts again is observed.

In addition, when the content ratio of highly water-absorbent polymers is increased, there is a problem that the highly water-absorbent polymers easily move due to impact, pressure, etc. when the product is transported, and the moved highly water-absorbent polymers accumulate in a folded part of a folded product. As a result, absorption capacity is reduced, and a height of swelling due to water absorption is locally increased, resulting in a reduced feeling of wearing.

Therefore, a main problem of the invention is to provide an absorbent body that is less likely to cause gel blocking, can improve a body fluid absorption speed and absorption capability, can prevent reversion, and does not impair a feeling of wearing, a method for manufacturing the same, and an absorbent article.

### Solution to Problem

To solve the above problem, as the invention according to claim 1, there is provided an absorbent body including a water-absorbent fiber stack including pulp fibers and a highly water-absorbent polymer, and a cover sheet covering at least a skin-facing surface of the water-absorbent fiber stack, in which a plurality of protruding portions protruding outward and a plurality of depressed recessed portions adjacent to the protruding portions are formed on a non-skin-facing surface of the cover sheet, and the water-absorbent fiber stack is stacked in the recessed portions but not in the protruding portions.

In the invention according to claim 1, since the water-absorbent fiber stack is stacked in the recessed portions of the cover sheet in which the protruding portions and the recessed portions are formed in the structure, the body fluid can be reliably absorbed in the recessed portions of the cover sheet in which the water-absorbent fiber stack is disposed, and liquid permeability is ensured such that the body fluid passes through the cover sheet in the thickness direction through the protruding portions in which the water-absorbent fiber stack is hardly disposed. In this way, even when the content ratio of the highly water-absorbent polymers is increased as the water-absorbent fiber stack, since liquid permeability is ensured through the protruding portions of the cover sheet, a liquid permeation failure due to gel blocking of the highly water-absorbent polymer rarely occurs, the body fluid absorption speed and the absorption capability can be improved, and the liquid rarely spills on the surface side so that reversion of the body fluid can be prevented.

In addition, since the water-absorbent fiber stack is disposed in the recessed portions defined by the protruding portions of the cover sheet, the highly water-absorbent polymers do not easily move, a state in which the highly water-absorbent polymers are uniformly dispersed and disposed can be maintained, and it is possible to prevent deterioration of the absorption capability and deterioration of the feeling of wearing due to an uneven distribution of the polymers.

As the invention according to claim 2, there is provided the absorbent body according to claim 1, in which the water-absorbent fiber stack is provided to have a thickness equal to or less than a height of an adjacent protruding portion.

In the invention according to claim 2, since the water-absorbent fiber stack is provided to have a thickness equal to or less than the height of the adjacent protruding portion, the body fluid can move to a lower layer side of the water-absorbent fiber stack by passing through the protruding portions of the cover sheet. Further, when a member excellent in diffusibility in the surface direction, for example, a member having a larger blending ratio of pulp fibers when compared to the water-absorbent fiber stack is disposed adjacent to the lower layer side of the water-absorbent fiber stack, the body fluid rapidly diffuses inside this member, and the body fluid is rapidly absorbed by the water-absorbent fiber stack adjacent to the inside of this member or an upper layer side.

As the invention according to claim 3, there is provided the absorbent body according to any one of claims 1 or in which a rib-groove structure, in which the protruding portions are a plurality of ribs that extends along a longitudinal direction of the cover sheet and is disposed at intervals in a lateral direction and the recessed portions are grooves, is included.

In the invention according to claim 3, since the surface of the cover sheet facing the water-absorbent fiber stack has the rib-groove structure, the water-absorbent fiber stack is easily disposed along the groove, the water-absorbent fiber stack disposed in the groove is hard to move, and fixability of the water-absorbent fiber stack becomes excellent. In addition, the protruding portions serving as the ribs do not substantially contain the polymer, and thus serve as slits, and the body fluid easily diffuses along a direction in which the ribs extend.

As the invention according to claim 4, there is provided the absorbent body according to any one of claims 1 to 3, in which a second water-absorbent fiber stack having a blending ratio of pulp fibers set to be larger than a blending ratio of the water-absorbent fiber stack is arranged adjacent to a side of a non-skin-facing surface of the water-absorbent fiber stack, and at least a non-skin-facing surface of the second water-absorbent fiber stack is covered with a second cover sheet.

In the invention according to claim 4, since the second water-absorbent fiber stack having a blending ratio of pulp fibers set to be larger than a blending ratio of the water-absorbent fiber stack is arranged adjacent to a side of a non-skin-facing surface of the water-absorbent fiber stack, the body fluid moving to the lower layer side of the water-absorbent fiber stack through the protruding portions of the cover sheet can diffuse in a wide range inside the second water-absorbent fiber stack, a liquid permeation failure by gel blocking of the water-absorbent fiber stack can be more reliably prevented, and the body fluid absorption speed and the absorption capability can be further improved.

As the invention according to claim 5, there is provided a method for manufacturing the absorbent body according to any one of claims 1 to 4, the method including
preparing a cover sheet having the protruding portions and the recessed portions formed on one side surface in advance,
supplying the cover sheet with the surface on which the protruding portions and the recessed portions are formed facing an outer surface side to a stacking device including a stacking recessed portion having a predetermined shape on a peripheral surface, and
stacking the water-absorbent fiber stack on the cover sheet in the stacking recessed portion.

In the invention according to claim 5, since the cover sheet having the protruding portions and the recessed portions formed in advance is supplied to the stacking device with the surface on which the protruding portions and the recessed portions are formed facing the outer surface side, and the water-absorbent fiber stack is stacked on the cover sheet in the stacking recessed portion, the water-absorbent fiber stack is easily stacked in the recessed portions of the cover sheet.

As the invention according to claim 6, there is provided the method for manufacturing the absorbent body according to claim 5, further including performing a process of leveling a surface of the water-absorbent fiber stack in the stacking recessed portion after the water-absorbent fiber stack is stacked on the cover sheet in the stacking recessed portion.

In the invention according to claim 6, by performing the process of leveling the surface of the water-absorbent fiber stack in the stacking recessed portion using a scuffing roll, etc. after the water-absorbent fiber stack is stacked on the cover sheet in the stacking recessed portion, it is possible to reliably fix the water-absorbent fiber stack in the recessed portions, a part of the highly water-absorbent polymer is buried in the protruding portions, and it is possible to set a stacking height of the water-absorbent fiber stack to be equal or less than the height of the protruding portions.

As the invention according to claim 7, there is provided the method for manufacturing the absorbent body according to claim 5 or 6, the absorbent body according to claim 4 being manufactured, the method further including, in separate processes
stacking the second water-absorbent fiber stack by a second stacking device including a second stacking recessed portion having a predetermined shape on a peripheral surface and placing the second water-absorbent fiber stack on the supplied second cover sheet, and
disposing the water-absorbent fiber stack stacked on the cover sheet adjacent to the second water-absorbent fiber stack.

The invention according to claim 7 is a method for manufacturing the absorbent body in which the second water-absorbent fiber stack and the second cover sheet are stacked in this order adjacent to the non-skin-facing surface side of the water-absorbent fiber stack. In this case, in separate processes, a member obtained by placing the second water-absorbent fiber stack on the second cover sheet is manufactured, and the water-absorbent fiber stack stacked on the cover sheet is disposed adjacent to an upper surface of the second water-absorbent fiber stack.

As the invention according to claim 8, there is provided an absorbent article characterized in that the absorbent body according to any one of claims 1 to 4 is disposed between a liquid-permeable face sheet covering a side of a skin-facing surface and a leakage prevention sheet covering a side of a non-skin-facing surface.

### Advantageous Effects of the Invention

As described in detail above, according to the invention, it is possible to provide an absorbent body that is less likely to cause gel blocking, can improve a body fluid absorption speed and absorption capability, can prevent reversion, and does not impair a feeling of wearing, a method for manufacturing the same, and an absorbent article.

### Brief Description of the Drawings

Fig. 1 is a partially broken development diagram of an incontinence pad 1 according to the invention.
Fig. 2 is a diagram taken along the line II-II of Fig. 1.
Fig. 3 is a diagram taken along the line III-III of Fig. 1.
Fig. 4 is a plan view of a stacked body of a water-absorbent fiber stack 8 and a cover sheet 9 as viewed from a non-skin-facing surface side.
Fig. 5 is a diagram taken along the line V-V of Fig. 4.
Fig. 6 is a transverse cross-sectional view of an absorbent body 4 illustrating an absorption path of a body fluid.
Fig. 7 is a vertical cross-sectional view illustrating manufacturing apparatuses 30 and 40 of the absorbent body 4.
Fig. 8 is a vertical cross-sectional view illustrating the manufacturing apparatus 30.

### Mode for Carrying Out the Invention

Hereinafter, embodiments of the invention will be described in detail with reference to the drawings.

### <Examples of basic structure of incontinence pad>

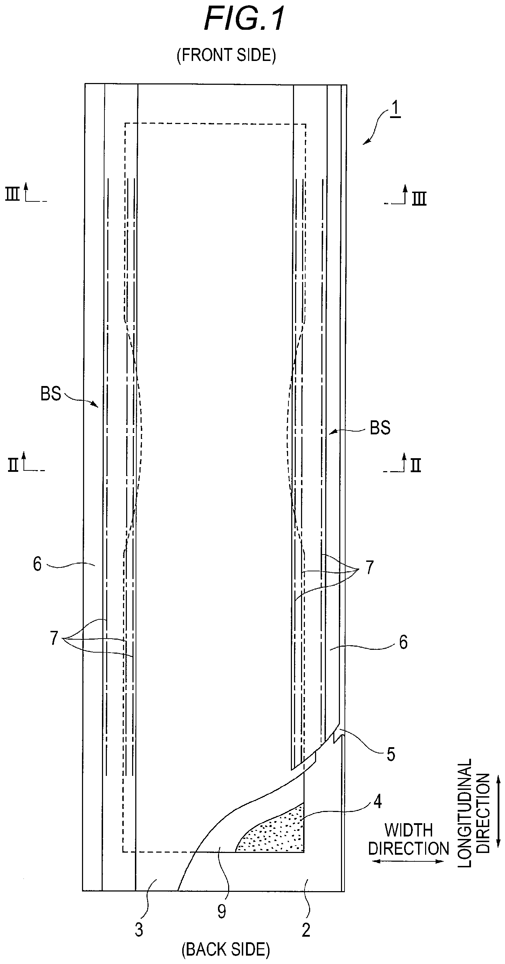

As illustrated in Fig. 1 to Fig. 3, an incontinence pad 1 of the invention mainly includes a leakage prevention sheet 2 made of a polyethylene sheet, etc., a face sheet 3 that forms a skin-contact surface and rapidly transmits urine, etc., an absorbent body 4 interposed between these sheets 2 and 3, an outer sheet 5 that covers an outermost surface (non-skin-contact surface) of the incontinence pad 1, and side nonwoven fabrics 6 and 6 using substantially side edge portions of the absorbent body 4 as standing base edges and forming a pair of left and right three-dimensional gathers BS and BS provided to protrude to a skin side within a predetermined section in a front-back direction including at least a urinating part of a wearer. Further, around the absorbent body 4, at upper and lower end edge portions thereof, outer edge portions of the leakage prevention sheet 2, the face sheet 3, and the outer sheet 5 are bonded by bonding means such as an adhesive such as hot melt, heat seal, or ultrasonic seal. In addition, at both side edge portions thereof, the leakage prevention sheet 2, the face sheet 3, the outer sheet 5, and the side nonwoven fabric 6 laterally extending from the absorbent body 4 are bonded by bonding means such as an adhesive such as hot melt, heat seal, or ultrasonic seal.

As the leakage prevention sheet 2, a sheet material having at least a water-blocking property such as polyethylene is used. However, in recent years, a sheet having moisture permeability tends to be used from a viewpoint of preventing stuffiness. As this water blocking and moisture-permeable sheet material, a microporous sheet obtained by melt kneading an inorganic filler in an olefin resin such as polyethylene or polypropylene to form a sheet, and then monoaxially or biaxially stretching the sheet is preferably used. As the leakage prevention sheet 2, a poly-laminated nonwoven fabric obtained by laminating a plastic film and a nonwoven fabric may be used.

Next, as the face sheet 3, a perforated or non-perforated nonwoven fabric, a porous plastic sheet, etc. is preferably used. As a material fiber included in the nonwoven fabric, it is possible to use a synthetic fiber such as an olefin-based fiber such as polyethylene or polypropylene, a polyester-based fiber, or a polyamide-based fiber, a regenerated fiber such as rayon or cupra, and a natural fiber such as cotton, and it is possible to use a nonwoven fabric obtained by an appropriate processing method such as a spun lace method, a spun bond method, a thermal bond method, a melt blown method, or a needle punch method. Among these processing methods, the spun lace method is excellent in flexibility and drapability, and the thermal bond method is excellent in bulkiness and high compression recovery. When a large number of through-holes are formed in the face sheet 3, the body fluid is quickly absorbed, and a dry touch property is excellent. A fiber of the nonwoven fabric may be a long fiber or a short fiber, and the short fiber is preferably used to give texture of a towel cloth. In addition, to facilitate an embossing treatment, it is preferable to use an olefin-based fiber having a relatively low melting point such as polyethylene or polypropylene. In addition, it is possible to preferably use a composite fiber of a core-sheath type fiber having a fiber having a high melting point as a core and a fiber having a low melting point as a sheath, a side-by-side type fiber, or a split type fiber.

The outer sheet 5 covers the leakage prevention sheet 2 to make an outer surface of the incontinence pad 1 look and feel like a cloth. The outer sheet 5 is preferably formed using a nonwoven fabric. As a material fiber, it is possible to use a synthetic fiber such as an olefin-based fiber such as polyethylene or polypropylene, a polyester-based fiber, or a polyamide-based fiber, a regenerated fiber such as rayon or cupra, and a natural fiber such as cotton. As a processing method, it is possible to use a spun lace method, a spun bond method, a thermal bond method, an air through method, a needle punch method, etc. However, in terms of achieving both texture and strength, it is preferable to use a long-fiber nonwoven fabric such as a spun bond nonwoven fabric, an SMS nonwoven fabric, or SMMS nonwoven fabric.

The nonwoven fabric may be used as a single sheet, or may be used by stacking a plurality of sheets. When the plurality of sheets is stacked and used, it is preferable that the nonwoven fabrics are fixed to each other via an adhesive such as hot melt. In addition, when a nonwoven fabric is used, a fiber basis weight thereof is preferably 10 to 50 g/m², and particularly preferably 15 to 30 g/m².

One or a plurality of temporary fixing layers (not illustrated) may be provided on a non-use surface side (outer surface) of the outer sheet 5, and the incontinence pad 1 may be fixed to underwear, a disposable diaper, etc. during wearing on a body. As the temporary fixing layer, a mechanical bonding type hook material may be used, or an adhesive may be used. The temporary fixing layer is provided as needed and may not be provided in particular.

In the illustrated example, the outer sheet 5 is made slightly wider than a width of the absorbent body 4, and both side portions are extended to a skin side of the face sheet 3 so that side edges of the leakage prevention sheet 2 and the face sheet 3 are rolled up. Further, on outer sides of the face sheet 3 in the width direction, side nonwoven fabrics 6 extending from surfaces of both side portions of the face sheet 3 to outer surface sides of the outer sheet 5 extending to the skin side, specifically, the side nonwoven fabrics 6 configured using a nonwoven fabric material subjected to an appropriate water repellent treatment or hydrophilic treatment to prevent penetration of urine or enhance texture are arranged. As such side nonwoven fabrics 6, it is possible to use those formed by an appropriate processing method using a natural fiber, a synthetic fiber, a regenerated fiber, etc. as a material. It is preferable to use a nonwoven fabric having a reduced basis weight and air permeability in order to eliminate a feeling of stiffness and prevent stuffiness. Specifically, it is preferable to use a nonwoven fabric having a basis weight of 13 to 23 g/m². Further, to prevent permeation of the body fluid, a water-repellent nonwoven fabric coated with a silicone-based, paraffin-based, or alkylchromic chloride-based water repellent is preferably used.

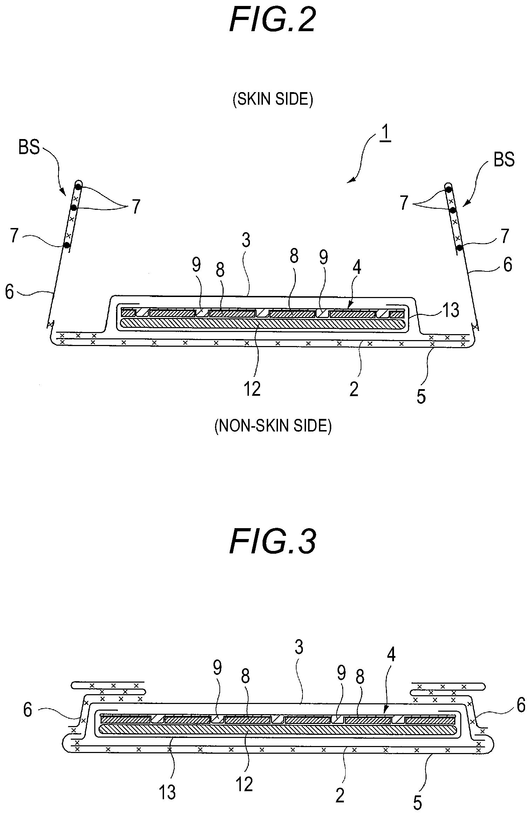

An inner side portion of each of the side nonwoven fabrics 6 is folded back substantially double, and one or a plurality of (three in the illustrated example) thread-shaped elastically stretchable members 7, 7, ... whose both ends or appropriate position in a longitudinal direction is fixed to an intermediate position in a height direction are arranged inside the double sheet in a state in which the both ends or the appropriate position in the longitudinal direction is fixed. At front and back end portions, as illustrated in Fig. 3, this double sheet portion is bonded to the absorbent body 4 side in a state of being folded back to the outer side once and laminated. In this way, at least within a predetermined section in the front-back direction including the urinating part of the wearer, as illustrated in Fig. 2, three-dimensional gathers BS and BS standing to the skin side are formed in a left-right pair.

### <Absorbent body>

The absorbent body 4 interposed between the leakage prevention sheet 2 and the face sheet 3 includes a water-absorbent fiber stack 8 containing a pulp fiber and a highly water-absorbent polymer and a cover sheet 9 covering at least a skin-facing surface of the water-absorbent fiber stack 8, and has a property of absorbing and retaining the body fluid.

For example, the water-absorbent fiber stack 8 includes cotton-like pulp and a highly water-absorbent polymer. The highly water-absorbent polymer is mixed in the pulp included in the water-absorbent fiber stack 8 as, for example, granular powder. Examples of the pulp include a pulp containing a cellulose fiber such as chemical pulp or dissolving pulp obtained from wood, or an artificial cellulose fiber such as rayon or acetate, and softwood pulp having a longer fiber length is more preferably used than hardwood pulp in terms of function and cost.

In addition, a synthetic fiber may be mixed with the water-absorbent fiber stack 8. As the synthetic fiber, for example, it is possible to use a polyolefin-based fiber such as polyethylene or polypropylene, a polyester-based fiber such as polyethylene terephthalate or polybutylene terephthalate, a polyamide-based fiber such as nylon, or a copolymer thereof, or it is possible to use a mixture of these two types. In addition, it is possible to use a composite fiber such as a core-sheath type fiber having a fiber having a high melting point as a core and a fiber having a low melting point as a sheath, a side-by-side type fiber, or a split type fiber. As the synthetic fiber, in the case of a hydrophobic fiber, it is desirable to use a synthetic fiber subject to surface treatment using a hydrophilizing agent to have an affinity for the body fluid.

The highly water-absorbent polymer includes "powder" in addition to "particles". As the highly water-absorbent polymer, it is possible to use those having a particle size used in this type of absorbent article without change, and it is desirable that those having an average particle size of 1,000 µm or less, preferably a particle size of 106 µm or more before absorption account for 99% by weight or more of the whole, and particularly those having 150 to 850 µm account for 99% by weight or more of the whole. The average particle size before absorption is preferably about 250 to 500 µm. In addition, it is desirable that an average particle size of the highly water-absorbent polymer after absorption is three times or more of the average particle size before absorption, specifically 500 µm or more. Note that the average particle size of the highly water-absorbent polymer before absorption means a particle size at an integrated value of 50% in a weight-based particle size distribution. The weight-based particle size distribution in this case is measured in accordance with JISZ8815-1994. In more detail, the weight-based particle size distribution is measured by stacking sieves having an inner diameter of 150 mm, a depth of 45 mm, and apertures of 710 µm, 500 µm, 300 µm, 150 µm, and 106 µm with a sieve having a narrower aperture on a lower side, putting 50 g of a measurement sample on the 710 µm sieve having a widest aperture at a top, performing sieving using a sieving machine for 10 minutes, measuring a weight of the measurement sample left on each sieve, and obtaining a weight% of the measurement sample left on each sieve based on a first weight of the measurement sample.

A basis weight amount of the highly water-absorbent polymer can be appropriately determined according to an absorption amount required for use of the absorbent body 4. Therefore, even though it cannot be generally stated, the basis weight amount can be set to 50 to 350 g/m². When the basis weight amount of the polymer is less than 50 g/m², it becomes difficult to ensure the absorption amount. When the basis weight amount exceeds 350 g/m², not only the effect is saturated, but also an excessively high amount of the highly water-absorbent polymer gives a gritty feeling.

In the water-absorbent fiber stack 8, a ratio of the highly water-absorbent polymer to a total weight of the pulp and the highly water-absorbent polymer is preferably 50 to 90% by weight. As described in detail later, since the water-absorbent fiber stack 8 is disposed in a predetermined region of the cover sheet 9, when the ratio of the highly water-absorbent polymer is less than 50% by weight, there is concern that the absorption capability may be insufficient. On the other hand, when the ratio is larger than 90% by weight, the gritty feeling (particle feeling) of the highly water-absorbent polymer becomes stronger, and usability deteriorates.

The cover sheet 9 is formed using tissue paper, especially crepe paper, a nonwoven fabric, a poly-laminated nonwoven fabric, a sheet having small holes, etc. However, it is desirable that the sheet does not allow the highly water-absorbent polymer to escape. When the nonwoven fabric is used in place of the crepe paper, an air through nonwoven fabric easily having a bulky structure and excellent in irregularity processing suitability is particularly preferable. The fiber basis weight is preferably 15 g/m² or more, and particularly preferably 20 to 100 g/m² so that concaves and convexes described later are easily formed.

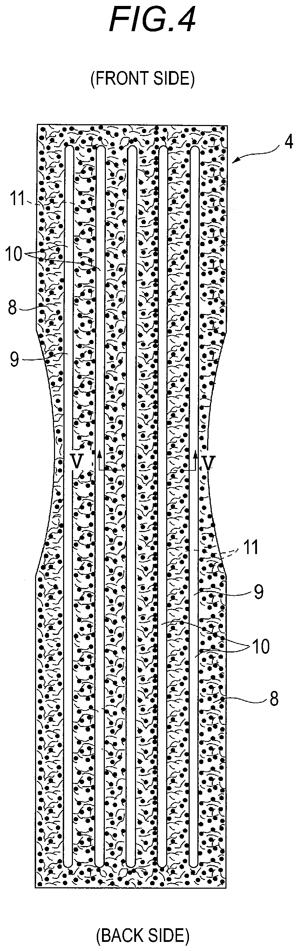

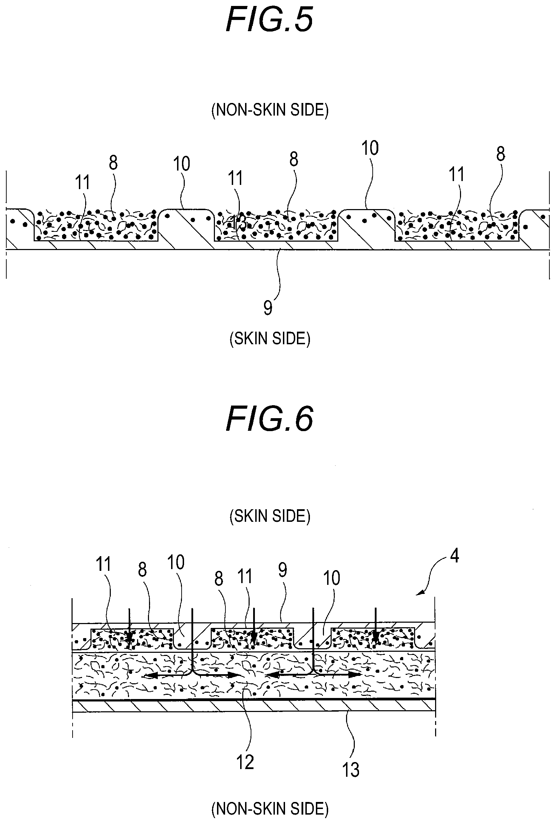

As illustrated in Fig. 4 and Fig. 5, on a non-skin-facing surface of the cover sheet 9, a plurality of protruding portions 10 protruding to the outer side (non-skin side) and depressed recessed portions 11 adjacent to the protruding portions 10 are formed. That is, concaves and convexes having different heights in a thickness direction are formed by the protruding portions 10 and the recessed portions 11 only on a surface (non-skin-facing surface) opposite to a surface on a side facing a skin surface of a wearer when the incontinence pad 1 is worn in the cover sheet 9, and a surface (skin-facing surface) on the opposite side is a flat surface.

As illustrated in Fig. 4, the protruding portions 10 are preferably provided in an intermediate portion which does not reach the outer edge of the absorbent body 4, and the recessed portions 11 are preferably formed at the outer edge thereof. In the plan view illustrated in Fig. 4, the area occupied by the protruding portions 10 is 50% or less, preferably 10 to 35% of the area of the absorbent body 4 (the total area of the protruding portions 10 and the recessed portions 11) . As will be described in detail later, since the water-absorbent fiber stack 8 is disposed in the recessed portions 11, when the area of the protruding portions 10 is excessively large, there is concern that the absorption capability may deteriorate.

The cover sheet 9 extends almost to the outer edge of the absorbent body 4, the water-absorbent fiber stack 8 is disposed in a predetermined region with a predetermined thickness, and the cover sheet 9 and the water-absorbent fiber stack 8 are included in an integral stacked body.

In the recessed portions 11, the water-absorbent fiber stack 8 is stacked on the surface on which the concaves and convexes are formed. It is preferable that the water-absorbent fiber stack 8 is stacked in the recessed portions 11 of the cover sheet 9 and is hardly stacked in the protruding portions 10. Not being stacked in the protruding portions 10 means that the pulp fibers and the highly water-absorbent polymer included in the water-absorbent fiber stack 8 are substantially absent in the protruding portions 10, and refers to a state in which the pulp fibers and the highly water-absorbent polymer are not present at all or slightly present by performing a suction treatment during stacking or a treatment for leveling the surface using a scuffing roll, etc. even though the amount is significantly small when compared to the recessed portions 11.

The water-absorbent fiber stack 8 is preferably provided to have a thickness equal to or less than the height of the adjacent protruding portion 10. That is, it is preferable that the water-absorbent fiber stack 8 is stacked with respect to the recessed portions 11 at substantially the same height as or lower than that of the protruding portions 10.

In the absorbent body 4, as illustrated in Fig. 2 and Fig. 6, it is desirable that a second water-absorbent fiber stack 12 including pulp fibers and a highly water-absorbent polymer are arranged adjacent to the non-skin-facing surface side of the water-absorbent fiber stack 8, and at least a non-skin-facing surface of the second water-absorbent fiber stack 12 is covered with a second cover sheet 13. In this instance, it is preferable that a blending ratio of the pulp fibers is set to be large, and a blending ratio of the highly water-absorbent polymer is set to be small in the second water-absorbent fiber stack 12 than in the water-absorbent fiber stack 8. Specifically, it is preferable that a ratio of the pulp fibers to the total weight of the pulp and highly water-absorbent polymer is 50 to 90% by weight, preferably 60 to 90% by weight. By including a large amount of pulp fibers in the second water-absorbent fiber stack 12 as described above, rapid diffusion in a plane direction due to a capillary action is likely to occur when the body fluid is absorbed.

The second water-absorbent fiber stack 12 is formed in a plane shape that is substantially the same as a stacked body of the cover sheet 9 and the water-absorbent fiber stack 8.

Similarly to the cover sheet 9, the second cover sheet 13 can be formed using tissue paper, especially crepe paper, a nonwoven fabric, a poly-laminated nonwoven fabric, a sheet having small holes, etc. However, it is desirable that the sheet does not allow the highly water-absorbent polymer to escape. When the nonwoven fabric is used in place of the crepe paper, a hydrophilic SMMS (spun bond/melt blown/melt blown/spun bond) nonwoven fabric is particularly preferable, and polypropylene, polyethylene/polypropylene, etc. can be used as a material thereof. A fiber basis weight is preferably 5 to 40 g/m², and particularly preferably 10 to 30 g/m².

The second cover sheet 13 is substantially the same as or slightly longer than the stacked body of the cover sheet 9 and the water-absorbent fiber stack 8 in a pad longitudinal direction, and is stacked in a size that substantially matches with both ends of the stacked body or set to be slightly longer than the stacked body in the longitudinal direction. On the other hand, as illustrated in Fig. 2, in a pad width direction, the second cover sheet 13 is provided to extend to both sides of the stacked body of the cover sheet 9 and the water-absorbent fiber stack 8 and folded at both side portions on the skin-facing surface side of the cover sheet 9 so that side edges of the stacked body and the second water-absorbent fiber stack 12 are rolled up. In this way, both side edges of the absorbent body 4 are covered with the second cover sheet 13, so that lateral leakage rarely occurs in the structure.

As described above, since the water-absorbent fiber stack 8 is stacked in the recessed portions 11 of the cover sheet 9 in which the protruding portions 10 and the recessed portions 11 are formed in the structure, as illustrated in Fig. 6, the body fluid can be reliably absorbed in the recessed portions 11 of the cover sheet 9 in which the water-absorbent fiber stack 8 is disposed, and liquid permeability is ensured such that the body fluid passes through the cover sheet 9 in the thickness direction through the protruding portions 10 in which the water-absorbent fiber stack 8 is hardly disposed. In this way, even when the content ratio of the highly water-absorbent polymers is increased as the water-absorbent fiber stack 8, since liquid permeability is ensured through the protruding portions 10 of the cover sheet 9, a liquid permeation failure due to gel blocking of the highly water-absorbent polymer rarely occurs, the body fluid absorption speed and the absorption capability can be improved, and the liquid rarely spills on the surface side so that reversion of the body fluid can be prevented.

In addition, since the water-absorbent fiber stack 8 is disposed in the recessed portions 11 defined by the protruding portions 10 of the cover sheet 9, the highly water-absorbent polymers do not easily move, a state in which the highly water-absorbent polymers are uniformly dispersed and disposed can be maintained, and it is possible to prevent deterioration of the absorption capability and deterioration of the feeling of wearing due to an uneven distribution of the polymers.

In addition, since the water-absorbent fiber stack 8 is provided to have a thickness equal to or less than a height of the adjacent protruding portion 10, the body fluid easily moves to the second water-absorbent fiber stack 12 disposed on a lower layer side of the water-absorbent fiber stack 8 by passing through the protruding portions 10 of the cover sheet 9.

Further, as illustrated in Fig. 6, since the second water-absorbent fiber stack 12 excellent in diffusibility in the surface direction is disposed adjacent to the lower layer side of the water-absorbent fiber stack 8, the body fluid rapidly diffuses inside the second water-absorbent fiber stack 12, and the body fluid is rapidly absorbed by the water-absorbent fiber stack 8 adjacent to an inside of this member or an upper layer side.

The protruding portions 10 and the recessed portions 11 can be disposed in any pattern. An arrangement pattern of Figs. 4 and 5 has a rib-groove structure in which the protruding portions 10 are a plurality of ribs that extends along the longitudinal direction of the cover sheet 9 and is disposed at intervals in a lateral direction and the recessed portions 11 are grooves. When the cover sheet 9 has the rib-groove structure, the water-absorbent fiber stack 8 can be easily disposed along the groove, the water-absorbent fiber stack 8 disposed in the groove is hard to move, and fixability of the water-absorbent fiber stack 8 becomes excellent. In addition, the protruding portions 10 serving as the ribs do not substantially contain the highly water-absorbent polymer or contain a small amount of the highly water-absorbent polymer, if any, and thus serve as slits in which the highly water-absorbent polymer is not present, and the body fluid easily diffuses along a direction in which the ribs extend. The rib-groove structure is formed linearly in the longitudinal direction at substantially equal intervals in the width direction of the absorbent body 4. However, to improve liquid permeability in the urinating part, the rib-groove structure may be radially disposed such that an interval between adjacent protruding portions 10 and 10 is smallest in the urinating part and gradually widens in the front-back direction.

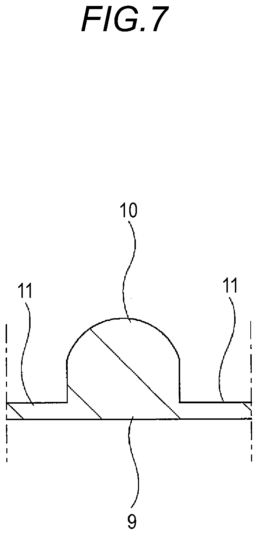

Upper surfaces (non-skin-facing surfaces) of the protruding portions 10 are formed to be substantially flat in the example illustrated in Fig. 5. However, the surfaces are more preferably formed in curved surfaces bulged to the non-skin side in a cross-sectional view of the absorbent body 4 illustrated in Fig. 7. When apexes of the protruding portions 10 are set to curved surfaces bulged outward, the protruding portions 10 are likely to project to the outside of the water-absorbent fiber stack 8 stacked in the recessed portions 11 and likely to come into contact with the second water-absorbent fiber stack 12 on the lower layer side, and thus the body fluid on the skin side easily moves to the second water-absorbent fiber stack 12 through the protruding portions 10.

### <Method for manufacturing absorbent body>

Next, a description will be given of a method for manufacturing the absorbent body 4. The cover sheet 9 having the protruding portions 10 and the recessed portions 11 on one side surface is prepared in advance. A publicly known method can be used for producing the protruding portions 10 and the recessed portions 11. For example, the concaves and convexes may be provided by performing embossing on portions corresponding to the recessed portions 11 for a nonwoven fabric having a flat surface, or the concaves and convexes may be provided by blowing a fluid to the portions corresponding to the recessed portions 11 to gather fibers of the recessed portions 11 in the protruding portions 10 in a nonwoven fabric manufacturing process of the cover sheet 9.

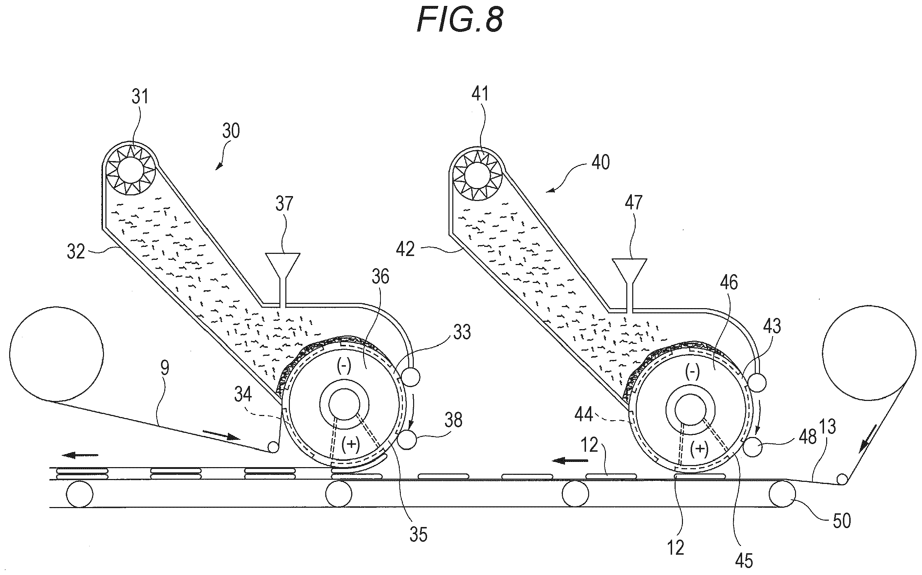

Subsequently, the stacked body of the cover sheet 9 and the water-absorbent fiber stack 8 is manufactured. For example, such a stacked body is manufactured by a first manufacturing apparatus 30 illustrated in Fig. 8. The first manufacturing apparatus 30 includes a defibrating device 31 for finely crushing the fiber sheet material included in the water-absorbent fiber stack 8 to be supplied, a crushed pulp supply casing 32 for surrounding the defibrating device 31 and for carrying the crushed pulp subjected to crushing on an air stream, and a stacking device 33 disposed in a downstream side opening of the crushed pulp supply casing 32. The stacking device 33 includes stacking recessed portions 34, 34, ... at an appropriate interval on an outer peripheral surface, includes a stacking rotary drum 35 having a large number of suction holes (not illustrated) in a bottom surface of the stacking recessed portion 34 and a suction chamber 36 arranged inside the stacking rotary drum 35, and an absorbent body component fiber air-transported into the stacking recessed portion 34 is stacked by maintaining a negative pressure in the suction chamber 36 by suction means (not illustrated). Note that a polymer input port 37 for supplying granules of the highly water-absorbent polymer together with the crushed pulp is provided in the crushed pulp supply casing 32.

To manufacture the stacked body of the cover sheet 9 and the water-absorbent fiber stack 8 by the first manufacturing apparatus 30, the cover sheet 9 in which the protruding portions 10 and the recessed portions 11 are formed is supplied to the stacking device 33 with the concave-convex forming surface facing outward, the cover sheet 9 is held by the stacking rotary drum 35 of the stacking device 33, and fibers defibrated by the defibrating device 31 are stacked on the cover sheet 9 in the stacking recessed portion 34.

In stacking of the water-absorbent fiber stack 8, the granules of the highly water-absorbent polymer supplied together with the crushed pulp are buried in each of the protruding portions 10 and the recessed portions 11 of the cover sheet 9 by being sucked from a bottom surface of the stacking recessed portion 34. In this way, the absorption capability is exhibited in the protruding portions 10, and the body fluid permeating the cover sheet 9 through the protruding portions 10 is also absorbed and held in the highly water-absorbent polymer.

In the water-absorbent fiber stack 8 deposited at a height equal to or higher than the height of the protruding portions 10, fibers accumulated on the protruding portions 10 are dropped into the recessed portions 11 by a scuffing roll 38 and are evened at the height of the protruding portions 10 as a whole. In this instance, the excess water-absorbent fiber stack 8 is scraped off by the scuffing roll 38.

Meanwhile, a stacked body of the second water-absorbent fiber stack 12 and the second cover sheet 13 is manufactured by a second manufacturing apparatus 40 illustrated in Fig. 8. An ordinary stacking device can be used as the second manufacturing apparatus 40. Such a second manufacturing apparatus 40 includes a defibrating device 41 for finely crushing the fiber sheet material included in the second water-absorbent fiber stack 12 to be supplied, a crushed pulp supply casing 42 for surrounding the defibrating device 41 and for carrying the crushed pulp subjected to crushing on an air stream, and a stacking device 43 arranged in a downstream side opening of the crushed pulp supply casing 42. The stacking device 43 includes stacking recessed portions 44 at an appropriate interval on an outer peripheral surface, includes a stacking rotary drum 45 having a large number of suction holes (not illustrated) in a bottom surface of the stacking recessed portion 44 and a suction chamber 46 arranged inside the stacking rotary drum 45, and an absorbent body component fiber air-transported into the stacking recessed portion 44 is stacked by maintaining a negative pressure in the suction chamber 46 by suction means (not illustrated). Note that a polymer input port 47 for supplying granules of the highly water-absorbent polymer together with the crushed pulp is provided in the crushed pulp supply casing 42. In addition, it is possible to include a scuffing roll 48 that scrapes off an excessive amount of the second water-absorbent fiber stack 12 accumulated to overflow the stacking recessed portion 44.

To manufacture the stacked body of the second water-absorbent fiber stack 12 and the second cover sheet 13 using such a second manufacturing apparatus 40, the second water-absorbent fiber stack 12 is manufactured by stacking using the stacking device 43 after defibrating using the defibrating device 41, and the second water-absorbent fiber stack 12 is transferred onto the second cover sheet 13 serving as a carrier sheet conveyed by a belt conveyor 50.

The second water-absorbent fiber stack 12 transferred onto the second cover sheet 13 is conveyed to the first manufacturing apparatus 30 by the belt conveyor 50, the stacked body of the cover sheet 9 and the water-absorbent fiber stack 8 manufactured by the first manufacturing apparatus 30 is placed, and the absorbent body 4 is completed.

### Reference Signs List

- 1: Incontinence pad
- 2: Leakage prevention sheet
- 3: Face sheet
- 4: Absorbent body
- 5: Outer sheet
- 6: Side nonwoven fabric
- 7: Thread-shaped elastically stretchable member
- 8: Water-absorbent fiber stack
- 9: Cover sheet
- 10: Protruding portion
- 11: Recessed portion
- 12: Second water-absorbent fiber stack
- 13: Second cover sheet
- 30: First manufacturing apparatus
- 31, 41: Defibrating device
- 32, 42: Crushed pulp supply casing
- 33, 43: Stacking device
- 34, 44: Stacking recessed portion
- 35, 45: Stacking rotary drum
- 36, 46: Suction chamber
- 37, 47: Polymer input port
- 38, 48: Scuffing roll
- 50: Belt conveyor

## Claims

1. An absorbent body (4) comprising:
a water-absorbent fiber stack (8) including pulp fibers and a highly water-absorbent polymer; and
a cover sheet (9) covering at least a skin-facing surface of the water-absorbent fiber stack,
**characterized in that** a plurality of protruding portions (10) protruding outward and a plurality of depressed recessed portions (11) adjacent to the protruding portions are formed on a non-skin-facing surface of the cover sheet, and the water-absorbent fiber stack is stacked in the recessed portions but not in the protruding portions.

2. The absorbent body according to claim 1, wherein the water-absorbent fiber stack is provided to have a thickness equal to or less than a height of an adjacent protruding portion.

3. The absorbent body according to any one of claims 1 to 2, wherein a rib-groove structure, in which the protruding portions are a plurality of ribs that extends along a longitudinal direction of the cover sheet and is disposed at intervals in a lateral direction and the recessed portions are grooves, is included.

4. The absorbent body according to any one of claims 1 to 3, wherein a second water-absorbent fiber stack (12) having a blending ratio of pulp fibers set to be larger than a blending ratio of the water-absorbent fiber stack is arranged adjacent to a side of a non-skin-facing surface of the water-absorbent fiber stack, and at least a non-skin-facing surface of the second water-absorbent fiber stack is covered with a second cover sheet.

5. A method for manufacturing the absorbent body according to any one of claims 1 to 4, the method comprising:
preparing a cover sheet (9) having the protruding portions (10) and the recessed portions (11) formed on one side surface in advance;
supplying the cover sheet with the surface on which the protruding portions and the recessed portions are formed facing an outer surface side to a stacking device including a stacking recessed portion having a predetermined shape on a peripheral surface; and
stacking the water-absorbent fiber stack (8) on the cover sheet in the stacking recessed portion.

6. The method for manufacturing the absorbent body according to claim 5, further comprising
performing a process of leveling a surface of the water-absorbent fiber stack in the stacking recessed portion after the water-absorbent fiber stack is stacked on the cover sheet in the stacking recessed portion.

7. The method for manufacturing the absorbent body according to claim 5 or 6, the absorbent body according to claim 4 being manufactured, the method further comprising, in separate processes:
stacking the second water-absorbent fiber stack by a second stacking device including a second stacking recessed portion having a predetermined shape on a peripheral surface and placing the second water-absorbent fiber stack on the supplied second cover sheet; and
disposing the water-absorbent fiber stack stacked on the cover sheet adjacent to the second water-absorbent fiber stack.

8. An absorbent article **characterized in that** the absorbent body according to any one of claims 1 to 4 is disposed between a liquid-permeable face sheet covering a side of a skin-facing surface and a leakage prevention sheet covering a side of a non-skin-facing surface.

## Patentansprüche

1. Absorbierender Körper (4), umfassend:
einen wasserabsorbierenden Faserstapel (8), darin eingeschlossen Pulpenfasern und ein stark wasserabsorbierendes Polymer; und
eine Deckschicht (9), die mindestens eine auf die Haut gerichtete Fläche des wasserabsorbierenden Faserstapels bedeckt,
**dadurch gekennzeichnet, dass** eine Vielzahl von vorspringenden Abschnitten (10) nach außen vorspringt und eine Vielzahl von versenkten, ausgesparten Abschnitten (11) benachbart den vorspringenden Abschnitten auf einer nicht auf die Haut gerichteten Fläche der Deckschicht gebildet ist und der wasserabsorbierende Faserstapel in den ausgesparten Abschnitten gestapelt ist, jedoch nicht in den vorspringenden Abschnitten.

2. Absorbierender Körper nach Anspruch 1 wobei der wasserabsorbierende Faserstapel bereitgestellt ist, um eine Dicke aufzuweisen, die gleich wie oder geringer als eine Höhe eines benachbarten vorspringenden Abschnitts ist.

3. Absorbierender Körper nach einem der Ansprüche 1 bis 2, wobei eine Rippen-Nut-Struktur, bei der die vorspringenden Abschnitte eine Vielzahl von Rippen sind, die sich entlang einer Längsrichtung der Deckschicht erstrecken, und in Intervallen in einer seitlichen Richtung angeordnet sind, und die ausgesparten Abschnitte Nuten sind, eingeschlossen ist.

4. Absorbierender Körper nach einem der Ansprüche 1 bis 3, wobei ein zweiter wasserabsorbierender Faserstapel (12), der ein Mischungsverhältnis von Pulpenfasern aufweist, das eingestellt ist, um grösser als ein Mischverhältnis des wasserabsorbierenden Faserstapels zu sein, benachbart einer Seite einer nicht auf die Haut gerichteten Fläche des wasserabsorbierenden Faserstapels angeordnet ist, und mindestens eine nicht auf die Haut gerichtete Fläche des zweiten wasserabsorbierenden Faserstapels mit einer zweiten Deckschicht bedeckt ist.

5. Verfahren zum Herstellen des absorbierenden Körpers nach einem der Ansprüche 1 bis 4, wobei das Verfahren Folgendes umfasst:
Vorbereiten einer Deckschicht (9), deren vorspringende Abschnitte (10) und ausgesparte Abschnitte (11), auf einer der Seitenflächen im Voraus gebildet sind;
Zuführen der Deckschicht mit der Fläche, auf der die vorspringenden Abschnitte und die ausgesparten Abschnitte gebildet sind, die auf eine Seite der äußeren Fläche gerichtet sind, in eine Stapelvorrichtung, darin eingeschlossen einem ausgesparten Stapelabschnitt, der eine vorbestimmten Form auf einer Umfangsfläche aufweist; und
Stapeln des wasserabsorbierenden Faserstapels (8) auf der Deckschicht im ausgesparten Stapelabschnitt.

6. Verfahren zur Herstellung des absorbierenden Körpers nach Anspruch 5, weiter umfassend:
Durchführen eines Prozesses des Nivellierens einer Fläche des wasserabsorbierenden Faserstapels im ausgesparten Stapelabschnitt, nachdem der wasserabsorbierende Faserstapel auf der Deckschicht im ausgesparten Stapelabschnitt gestapelt ist.

7. Verfahren zur Herstellung des absorbierenden Körpers nach Anspruch 5 oder 6, wobei der absorbierende Körper nach Anspruch 4 hergestellt ist, wobei das Verfahren weiter in getrennten Prozessen Folgendes umfasst:
Stapeln des zweiten wasserabsorbierenden Faserstapels durch eine zweite Stapelvorrichtung, darin eingeschlossen einen zweiten ausgesparten Stapelabschnitt, der eine vorbestimmte Form auf einer Umfangsfläche aufweist, und Platzieren des zweiten wasserabsorbierenden Faserstapels auf dem zugeführten zweiten Deckschicht; und
Anordnen des gestapelten wasserabsorbierenden Faserstapels auf der Deckschicht benachbart dem zweiten wasserabsorbierenden Faserstapel.

8. Absorbierender Artikel, **dadurch gekennzeichnet, dass** der absorbierende Körper nach einem der Ansprüche 1 bis 4 zwischen einer flüssigkeitsdurchlässigen vorderen Schicht, die eine Seite einer auf die Haut gerichteten Fläche bedeckt, und einer Auslaufschutzschicht angeordnet ist, die eine Seite einer nicht auf die Haut gerichteten Fläche bedeckt.

## Revendications

1. Corps absorbant (4) comprenant :
une pile de fibres hydro-absorbantes (8) incluant des fibres de pâte à papier et un polymère hautement hydro-absorbant ; et
une feuille de couverture (9) couvrant au moins une surface orientée vers la peau de la pile de fibres hydro-absorbantes,
**caractérisé en ce qu'**une pluralité de parties en saillie (10) faisant saillie vers l'extérieur et une pluralité de parties en retrait (11) enfoncées adjacentes aux parties en saillie sont formées sur une surface non orientée vers la peau de la feuille de couverture, et la pile de fibres hydro-absorbantes est empilée dans les parties en retrait mais pas dans les parties en saillie.

2. Corps absorbant selon la revendication 1, dans lequel la pile de fibres hydro-absorbantes est prévue pour avoir une épaisseur inférieure ou égale à une hauteur d'une partie en saillie adjacente.

3. Corps absorbant selon l'une quelconque des revendications 1 à 2, dans lequel est incluse une structure de nervures-rainures, dans laquelle les parties en saillie sont une pluralité de nervures qui s'étendent le long d'une direction longitudinale de la feuille de couverture et sont disposées à des intervalles dans une direction latérale et les parties en retrait sont des rainures.

4. Corps absorbant selon l'une quelconque des revendications 1 à 3, dans lequel une seconde pile de fibres hydro-absorbantes (12) présentant un rapport de mélange de fibres de pâte à papier défini comme étant plus grand qu'un rapport de mélange de la pile de fibres hydro-absorbantes est agencée de manière adjacente à un côté d'une surface non orientée vers la peau de la pile de fibres hydro-absorbantes, et au moins une surface non orientée vers la peau de la seconde pile de fibres hydro-absorbantes est couverte avec une seconde feuille de couverture.

5. Procédé de fabrication du corps absorbant selon l'une quelconque des revendications 1 à 4, le procédé comprenant :
la préparation d'une feuille de couverture (9) ayant les parties en saillie (10) et les parties en retrait (11) formées sur une surface latérale à l'avance ;
la fourniture de la feuille de couverture avec la surface sur laquelle les parties en saillie et les parties en retrait sont formées orientées vers un côté de surface externe à un dispositif d'empilement incluant une partie en retrait d'empilement ayant une forme prédéterminée sur une surface périphérique ; et
l'empilement de la pile de fibres hydro-absorbantes (8) sur la feuille de couverture dans la partie en retrait d'empilement.

6. Procédé de fabrication du corps absorbant selon la revendication 5, comprenant en outre
la réalisation d'un processus de nivellement d'une surface de la pile de fibres hydro-absorbantes dans la partie en retrait d'empilement après que la pile de fibres hydro-absorbantes est empilée sur la feuille de couverture dans la partie en retrait d'empilement.

7. Procédé de fabrication du corps absorbant selon la revendication 5 ou 6, le corps absorbant selon la revendication 4 étant fabriqué, le procédé comprenant en outre, dans des processus distincts :
l'empilement de la seconde pile de fibres hydro-absorbantes par un second dispositif d'empilement incluant une seconde partie en retrait d'empilement ayant une forme prédéterminée sur une surface périphérique et le placement de la seconde pile de fibres hydro-absorbantes sur la seconde feuille de couverture fournie ; et
la disposition de la pile de fibres hydro-absorbantes empilée sur la feuille de couverture adjacente à la seconde pile de fibres hydro-absorbantes.

8. Article absorbant **caractérisé en ce que** le corps absorbant selon l'une quelconque des revendications 1 à 4 est disposé entre une feuille de face perméable aux liquides couvrant un côté d'une surface orientée vers la peau et une feuille de prévention de fuite couvrant un côté d'une surface non orientée vers la peau.
